# EUROPÄISCHE PATENTANMELDUNG

(11) **EP 1 790 288 A1**
(43) Veröffentlichungstag der Anmeldung: **30.05.2007**
(21) Anmeldenummer: 05025739.3
(22) Anmeldetag: 25.11.2005
(51) Int. Cl.: A61B 5/151

(54) **Geknickte Lanzette**

(71) Anmelder: Roche Diagnostics GmbH, 68305 Mannheim (DE); F.HOFFMANN-LA ROCHE AG, 4070 Basel (CH)
(72) Erfinder: Hoenes, Joachim, 64673 Zwingenberg (DE); Deck, Frank, 67150 Niederkirchen (DE); Haar, Hans-Peter, 69168 Wiesloch (DE); Kraemer, Uwe, 684549 Ilvesheim (DE); Zimmer, Volker, 67229 Laumershiem (DE)
(74) Vertreter: Poredda, Andreas

(57) **Zusammenfassung**

Es wird eine Vorrichtung zur Gewinnung von Körperflüssigkeiten beschrieben, die ein im Wesentlichen planares Trägerband mit einer Längsausrichtung und einer Querausrichtung besitzt, auf der mindestens eine Lanzette, beinhaltend einen Lanzettenkörper und eine Spitze angeordnet ist, wobei die Lanzette liegend auf dem Trägerband, angeordnet ist. Die Vorrichtung ist dadurch gekennzeichnet, dass die Lanzette eine Struktur mit veränderter Steifigkeit beinhaltet, die auch als Knickbereich bezeichnet werden kann. Bevorzugter weise besitzt diese Struktur erniedrigte Steifigkeit gegenüber dem restlichen Lanzettenkörper, so dass die Lanzette unter Krafteinwirkung bevorzugt in diesem Bereich geknickt wird. Dabei wird die Spitze in Bezug auf den restlichen Lanzettenkörper in ihrer Ausrichtung verändert. Diese Änderung der Ausrichtung ist bevorzugter weise aus der Trägerbandebene heraus.

## Beschreibung

### Technisches Gebiet

Die Erfindung liegt auf dem Gebiet der Stechhilfen zur diagnostischen Ermittelung von Blutparametem.

### Stand der Technik

Die Gewinnung und Analyse von Körperflüssigkeiten findet auf vielen Gebieten der medizinischen Diagnostik statt. Deshalb ist es wünschenswert auch Routinetests außerhalb des Laboratoriums schnell und reproduzierbar zu ermöglichen. Das Testen kann mit verschiedenen Körperflüssigkeiten durchgeführt werden, wie z. B. Blut und/oder interstitieller Flüssigkeit. Diese Flüssigkeiten können auf verschiedene Charakteristiken hin untersucht werden. Die Ergebnisse dieser Untersuchung sind wichtig, um verlässliche Diagnosen, therapeutische Maßnahmen und Therapieverfolgungen durchführen zu können.

Die Analyse von Körperflüssigkeiten beginnt mit der Gewinnung der Flüssigkeit. Eine Methode zur Gewinnung von Körperflüssigkeit besteht darin, eine minimale Wunde in die Haut des Patienten mit Hilfe einer Nadel, Lanzette oder eines Messers zu erzeugen. Die dabei gewonnene Körperflüssigkeit kann entweder in kleinen Gefäßen gesammelt werden oder direkt in Kontakt mit einem Testelement wie z. B. einem Teststreifen zur Analyse gebracht werden. Um bei der Benutzung der Lanzetten, Nadel oder Klingen eine Verletzungsgefahr des Patienten zu vermeiden, wird die Stechhilfe mit einem Schutz an der Lanzettenspitze konstruiert. Die meisten dieser Stechhilfen benötigen ein manuelles Einfügen der Lanzette in die Stechhilfe. Dies ist bei einer sehr häufigen Benutzung der Stechhilfe eine sehr umständliche Handhabung. Eine Magazinierung von Lanzetten könnte dieses Problem beheben, wobei hier viele Sicherheitsaspekte zu beachten sind. Es ist beispielsweise zu beachten, dass die Sicherheit des Patienten bei der Benutzung der Stechhilfe gewährleistet ist. Zudem sollte das System nicht zu komplex werden, da es sonst vom Patienten nicht gut handhabbar wäre.

Im Stand der Technik werden hierzu einige Lösungen aufgezeigt. Das US Patent 2003/0199902 gewährleistet eine Versiegelung jeder einzelnen Lanzette in einem Magazin, wobei ein aufwendiger und Raum erfüllender Zahnradmechanismus dazu benutzt wird, die Lanzetten aus dem Magazin zu befördern.

In der europäischen Anmeldung EP 1 203 563 wird ein analytisches Hilfsmittel beschrieben, das auf einem Trägerband ein Testelement aufweist, wobei auf diesem Testelement ein zusätzliches Rahmenelement aufgebracht ist, das beweglich ist und eine Lanzette beinhaltet. Das Rahmenelement kann bei Benutzung von einer parallelen Stellung zum Testelement in eine orthogonale Stellung bewegt werden, so dass die Lanzette durch eine Öffnung im Testelement aktuiert werden kann. Dies ist eine recht aufwendige Realisierung von einer Kombination von Testelement und Lanzette, da viele Teile mechanisch bewegt werden müssen und das System in seiner funktionsfähigen Form viel Platzbedarf hat.

Eine weitere europäische Anmeldung mit der Nummer EP 1 360 935, beschreibt eine Anordnung von Lanzetten (hier als "Tester" bezeichnet), um Zugang zu flüssigen Proben zu erhalten. Die Lanzetten sind dabei seriell auf einem Band angeordnet, das auf seiner Oberseite eine Abdeckung der Lanzetten aufweist. Um die Lanzette zur Benutzung freizulegen, wird auch hier ein aufwendiges mechanisches System benutzt, da der gesamte Lanzettenkörper zunächst aus der Bandebene heraus bewegt werden muss, um die Lanzette benutzen zu können.

Dieser Stand der Technik weist diverse Nachteile auf. Es sind viele mechanische Schritte notwendig, um das einzelne Stechelement aus der Magazinierung, bei der die Lanzetten in einer seriellen Anordnung, also in der Trägerbandebene liegen, in eine Anordnung zu bewegen, bei der die Lanzette senkrecht zur Trägerbandebene angeordnet ist. Dies hat aufgrund der aufwendigen Mechanik zusätzlich den Nachteil, dass ein großer Platzbedarf für diese Mechanik benötigt wird. Ein weiterer Nachteil vieler Systeme aus dem Stand der Technik ist die aufwendige Entsiegelung der Lanzetten vor dem Stechvorgang.

Aus den Nachteilen des Standes der Technik ergibt sich folgende Aufgabe. Es soll eine Platz sparende, mit wenig mechanischem Aufwand benutzbare, magazinierte Stechhilfe zur Verfügung gestellt werden, die eine einfache Handhabung möglich macht.

Diese Aufgabe wird durch den Gegenstand der Erfindung, wie er in den unabhängigen Patentansprüchen charakterisiert wird, gelöst. Bevorzugte Ausführungsformen sind Gegenstand der abhängigen Ansprüche.

Gegenstand der Erfindung ist ein Trägerband auf dem bevorzugt eine Mehrzahl Lanzetten positioniert sind. Es wird eine Vorrichtung zur Gewinnung von Körperflüssigkeiten beschrieben, die ein im Wesentlichen planares Trägerband mit einer Längsausrichtung und einer Querausrichtung besitzt, auf der mindestens eine Lanzette, beinhaltend einen Lanzettenkörper und eine Spitze angeordnet ist, wobei die Lanzette liegend auf dem Trägerband, angeordnet ist. Die Vorrichtung ist dadurch gekennzeichnet, dass die Lanzette eine Struktur mit veränderter Steifigkeit (gegenüber dem restlichen Lanzettenmaterial) beinhaltet, die auch als Knickbereich bezeichnet werden kann. Die Steifigkeit soll hierbei als Maß für den Widerstand des Materials gegen elastische Verformung verstanden werden. Bevorzugter weise besitzt diese Struktur erniedrigte Steifigkeit gegenüber dem restlichen Lanzettenkörper, so dass die Lanzette unter Krafteinwirkung bevorzugt in diesem Bereich geknickt wird. Dabei wird die Spitze in Bezug auf den restlichen Lanzettenkörper in ihrer Ausrichtung verändert. Diese Änderung der Ausrichtung ist bevorzugter weise aus der Trägerbandebene heraus. Dabei bleibt der Lanzettenkörper mindestens zu einem Teil in der Trägerbandebene und ist daran fixiert. Die Kraft, die zur Veränderung der Ausrichtung der Lanzettenspitze benötigt wird, wird auch als Schwellenkraft bezeichnet. Diese Schwellenkraft soll so groß sein, dass sie die Veränderung der Ausrichtung der Lanzettenspitze bewirkt, ist dabei aber so dimensioniert, dass keine ungewollten Verformungen an Lanzette oder am Trägerband auftreten.

Die Übertragung der Kraft auf die Lanzette kann durch ein Knickelement, z.B. einem Stößel stattfinden, der auf die Lanzette gedrückt wird. Bei einer speziellen Ausführungsform mit mehr als einer Prägung, besteht die Möglichkeit die Kraft auf die Lanzette zu übertragen indem das Trägerband mit der Lanzette über den Stößel geleitet wird. Hier wirkt eine ausreichend große Kraft (Schwellenkraft) auf die Lanzette, um die Lanzettenspitze aus der Trägerbandebene herauszubewegen, wobei zumindest ein Rest des Lanzettenkörpers am Trägerband verbleibt.

In einer bevorzugten Ausführungsform ist das Knickelement zweiteilig. Hierbei wird die Lanzettenspitze durch den Stößel aus der Trägerbandebene herausbefördert, indem der Lanzettenkörper zusammen mit dem Trägerband durch den zweiten Teil des Knickelements an einer Bewegung in Richtung der Stößelbewegung gehindert wird. Dieser zweite Teil des Knickelements kann beispielsweise eine Sperre sein, die sich bevorzugter weise auf der dem Stößel gegenüberliegenden Seite des Trägerbandes befindet. Sowohl der Stößel als auch die Sperre können zusätzlich durch ein Regelelement gesteuert werden, sodass die Position des Knickes variiert werden kann. Auf diese Weise kann die Lanzette an verschiedenen Stellen geknickt werden und es entstehen unterschiedlich lange Spitzen zum Einstechen in die Haut. Die Kraftübertragung vom Stößel auf die Lanzette ist besonders leicht möglich, wenn der Spitzenbereich der Lanzette mit dem Trägerband nicht fest verbunden ist. Besonders bevorzugt für die Anordnung auf einem Trägerband ist dabei eine Flachlanzette.

Der Knickbereich der Lanzette, der auch außerhalb des Spitzenbereiches liegen kann, weist mindestens eine Struktur mit veränderter Steifigkeit auf. Diese mindestens eine Struktur mit veränderter Steifigkeit wird im Folgenden als Prägung bezeichnet. Die Prägung kann durch z. B. Stanzen oder Hämmern oder sonstige Metall bearbeitende Maßnahmen in oder auf die Lanzette gearbeitet werden. Die Steifigkeit kann also vorzugsweise durch die Variation der Geometrie eines Bauteils oder durch die Variation der Materialmenge im Bauteil eingestellt werden. Eine bevorzugte Ausführungsform beinhaltet mehr als eine Prägung in dem Knickbereich der Lanzette. Eine besonders bevorzugte Ausführungsform dieser Prägung ist eine Dreifachprägung im Knickbereich der Lanzette, die sich über mindestens einen Teil der Längsausdehnung der Lanzette erstreckt. Eine Prägung erstreckt sich dabei von dem distalen Ende der Lanzette in axialer Richtung in Richtung zum proximalen Ende der Lanzette. Die Länge der Prägung ist variabel. Diese Prägung kann von zwei Seiten in die Flachlanzette eingebracht sein. Diese unterschiedliche Richtung der Prägung bewirkt, dass die Lanzettenspitze in die entgegen gesetzte Richtung zum Lanzettenkörper abknickt.

Der erste Teil der ersten Prägung befindet sich im Spitzenbereich. Dieser erste Teil der Prägung kann auf den Spitzenbereich beschränkt sein, aber auch darüber hinausgehen. Eine zweite Prägung grenzt an das proximale Ende des ersten Teils der ersten Prägung in Richtung Seitenkante der Lanzette an. Eine dritte Prägung verläuft ebenfalls angrenzend an das proximale Ende des ersten Teils der ersten Prägung in Richtung der gegenüberliegenden Seitenkante der ersten Prägung der Lanzette. Die zweite und dritte Prägung werden von der gleichen Seite eingeprägt wie der erste Teil der ersten Prägung. Durch die Ausrichtung dieser Prägungen ist es möglich, mittels einer niedrigen Schwellenkraft auf die Lanzette, ein Abknicken der an die mindestens eine Prägung angrenzenden Flächen, zu bewirken. Hierdurch werden die abgeknickten Flächen in einem Winkel von vorzugsweise bis zu 100 ° aus der Trägerbandebene gehoben. Dadurch wird die Lanzettenspitze aus der Ebene des Bandes herausbewegt.

Das Material der Lanzette ist bevorzugter weise Metall, besonders bevorzugt ist Stahl. Die Lanzette kann aber auch aus anderen Materialien bestehen, die es sowohl ermöglichen, dass die Lanzette bei Krafteinwirkung knickbar ist und dabei genügend Steifigkeit besitzt, um bei Benutzung in die Haut einzudringen ohne ihre Form zu verändern. Außerdem sollte das Material der Gestalt sein, dass es an dem distalen Ende der Lanzette zu einer scharfen Spitze gearbeitet werden kann, da sonst beim Stich zu viel Schmerz generiert wird. Die Herstellung von Lanzetten im Allgemeinen ist im Stand der Technik hinreichend bekannt, wie beispielsweise in DE19604156 oder EP0565970.

Das Trägerband ist bevorzugter weise aus einer Plastikfolie gefertigt. Dies kann aber auch ein anderes flexibleres Material sein, wie z.B. in der Anmeldung US 20050245845 beschrieben. In einem integrierten System kann auf dem Trägerband zusätzlich mindestens ein Testelement angeordnet sein. Bevorzugt werden Lanzette und Testelement alternierend angeordnet. Die Lanzette kann sowohl diagonal, in Längsausrichtung wie auch in Querausrichtung auf dem Band angebracht sein. Eine mögliche Ausführungsform ist die Anordnung von Lanzette und Testelement in direkter Nachbarschaft. Auf diese Weise ist ein direkter Transfer von Flüssigkeit nach dem Stechvorgang auf das Testelement möglich, ohne dass das Band weiter bewegt werden muss.

Zur Aktuierung der Lanzette werden im Folgenden verschiedene Möglichkeiten beschrieben. Die Lanzette kann an ihrem proximalen Ende auf dem Trägerband so fixiert sein, dass ein Teil der Lanzette in Relation zum oder mit dem Trägerband bewegt werden kann, während das proximale Ende an mindestens einem Punkt mit dem Trägerband verbunden ist. Eine weitere bevorzugte Befestigung der Lanzette, ist die Fixierung des Lanzettenkörpers an dem Trägerband, wobei sich der Spitzenbereich vom Trägerband löst. Die gesteuerte Bewegung der Lanzette kann durch Bewegen des Trägerbandes oder durch Ergreifen der Lanzette mit einem Greifelement geschehen, wobei die Lanzettenspitze mit dem Trägerband aus der Ebene des Trägerbandes herausbewegt wird. Diese Bewegung kann mittels eines Antriebselementes durchgeführt werden, das senkrecht zur Trägerbandebene auf die Lanzette Kraft überträgt. Die Kraftübertragung findet durch ein Antriebselement statt, das z.B. ein Stößel sein kann oder ein Greifelement, welches die Lanzette an ihrem Lanzettenkörper greift und bewegt. Hierbei ist in einer bevorzugten Ausführungsform die Einstichtiefe des Blutentnahmegerätes frei wählbar. Zur Regulierung der Einstichtiefe wird die Bewegung der Lanzette durch ein variierbares Anschlagselement definiert, gegen das die Lanzette während des Einstichvorgangs anschlägt. In Abhängigkeit von der Position des Anschlagelementes wird auf diese Weise die Länge der Lanzettenspitze, die aus der Gehäuseöffnung austritt und somit die Einstichtiefe variiert. Das Anschlagselement kann beispielsweise in das Gehäuse integriert werden. Des Weiteren kann die Lanzette selbst als Anschlagselement dienen, wobei die Stechtiefe durch die Länge der abgeknickten Spitze definiert wird. Da die Lanzette in abgeknicktem Zustand in einem von 0° abweichenden Winkel zum Lanzettenkörper abgeknickt ist, kann der Lanzettenkörper eine Barriere für das weitere Eindringen der Lanzette in die Haut darstellen. So ist es möglich, Lanzetten mit mehreren Prägungen im Knickbereich zu benutzen, um durch die Wahl der Prägung, die zum Knicken benutzt wird, die Stechtiefe zu verändern.

Zum Antrieb der Lanzette können ballistische oder Kulissen geführte Mechanismen benutzt werden, die aus dem Stand der Technik bekannt sind, wie z.B. in DE19604156, EP0565970, US5318584 oder US4924879 beschrieben. Eine bevorzugte Ausführungsform für den Antrieb der Lanzette ist die freie Bewegung der Lanzette nach Kraftübertragung durch das Antriebselement, wie beispielsweise dem Stößel. In dieser Ausführungsform wird ein Impuls von dem Antriebselement auf die Lanzette übertragen und die Lanzette bewegt sich ohne weitere Führung durch das Antriebselement in Richtung Gehäuseöffnung. Die Bewegung der Lanzette kann dabei durch zusätzliche Elemente am Gehäuse geführt werden.

Zur hygienischen Verwendung des Systems wird die Lanzette mindestens im Spitzenbereich durch einen Sterilschutz geschützt. Bevorzugter weise ist die Lanzette über den ganzen Lanzettenkörper mit dieser Folie überdeckt. Die Folie erstreckt sich dabei auch über einen Teil des Trägerbandes und ist damit verbunden. Dieser Sterilschutz kann aus einer Polymerschicht bestehen, die nach der Verknüpfung der Lanzette mit dem Trägerband aufgebracht wird. Diese Polymerschicht wird beim Aufwenden der Schwellenkraft auf die Lanzettenspitze von der Lanzettenspitze zerstört oder durchstochen und legt die Lanzettenspitze frei. Alternativ kann der Sterilschutz vor Benutzung der Lanzette entfernt werden. Bevorzugter weise wird der Sterilschutz hierbei im Ganzen entfernt.

Gegenstand der Erfindung ist ebenfalls ein System zur Gewinnung von Körperflüssigkeit. Dieses System besteht bevorzugter weise aus einem Gehäuse, in dem ein im Wesentlichen planares Trägerband montiert ist, und mindestens einer Lanzette, die liegend auf dem Trägerband angeordnet ist. Das Gehäuse weist mindestens eine Öffnung auf, durch die die Lanzette bei Aktuation hindurch treten kann. Das im Wesentlichen planare Trägerband ist bevorzugter weise auf zwei Spulen aufgewickelt. Es können aber auch andere Bevorratungsmöglichkeiten zur Magazinierung der benutzten und unbenutzten Lanzetten eingesetzt werden. Bei Verwendung von zwei Spulen zur Magazinierung der Lanzetten befinden sich die unbenutzten Lanzetten auf der einen Spule und die benutzten Lanzetten auf der anderen Spule. Die Lanzetten bestehen aus einem Material, das weich genug ist, um auf dem Trägerband aufgewickelt zu werden, ohne dabei geknickt zu werden. Andererseits ist das Material der Lanzetten so stabil, dass die Lanzette bei Aktuation und Eintritt in die Haut nicht verformt wird. Alternativ werden die Lanzetten quer auf dem Trägerband angeordnet, so dass ein Biegen der Lanzette vermieden werden kann. Eine weitere Möglichkeit ein Biegen der unbenutzten Lanzetten zu vermeiden, ist die Wahl des Durchmessers der Spule auf dem die Lanzetten aufbewahrt werden, sodass die Lanzetten beim Aufrollen kaum gebogen werden.

Die Lanzette weist eine Lanzettenspitze auf, die sich am distalen Ende der Lanzette befindet. Im System befindet sich ein Knickelement, das auf die Lanzette so einwirkt, dass die Lanzettenspitze in Bezug auf den restlichen Lanzettenkörper in ihrer Ausrichtung veränderbar ist. In einer bevorzugten Ausführungsform kann das Knickelement bei Krafteinwirkung auf die Lanzette vor der Aktuation, den Ort der Krafteinwirkung auf den Lanzettenkörper regeln. Hierzu kann das Knickelement durch ein Regelelement gesteuert werden. Zur Kraftübertragung kann ein Stößel dienen.

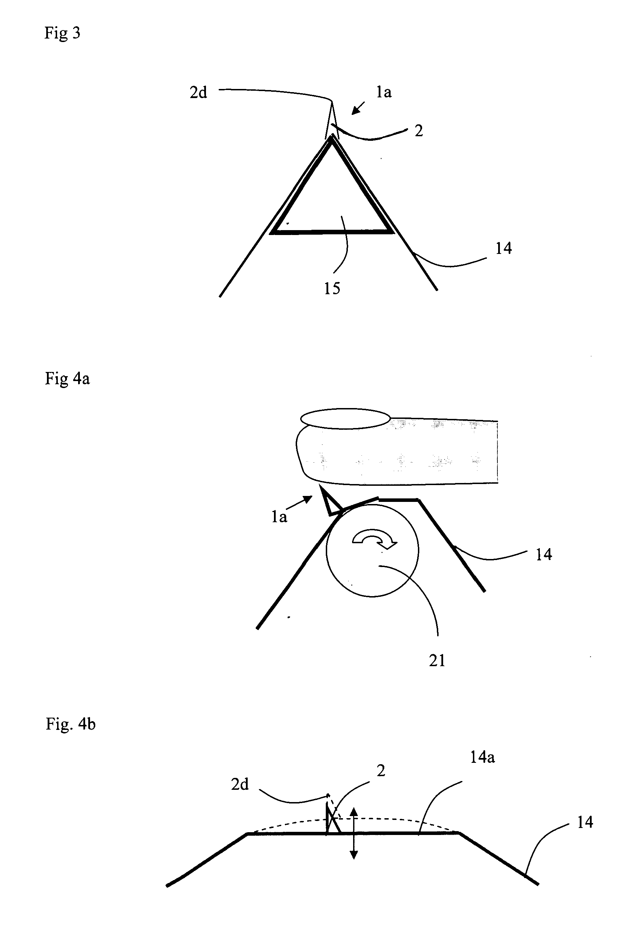

Eine weitere Ausführungsform des Knickelementes ist ein Stößel, über den das Trägerband mit der Lanzette geleitet wird, sodass die Kraft, die dabei auf die Lanzette wirkt ausreicht, um die Lanzettenspitze umzuknicken. Um eine gute Kraftübertragung auf die Lanzette zu erreichen, kann das Trägerband gespannt werden. Weitere Ausführungsformen für ein einstückiges Knickelement ist beispielsweise ein Rad mit einem möglichst kleinen Radius (s. Figur 4a), bzw. eine Führung über eine Kante (s. Figur 3), über die das Trägerband geleitet wird. Die Funktionsweise dieser Alternativen wird bei der Beschreibung der Figuren näher ausgeführt.

In dem System können herkömmliche Lanzetten benutzt werden, bevorzugter weise Flachlanzetten, aber auch alle Lanzetten, bei denen die Schwellenkraft des Knickelementes ausreicht, um die Spitze der Lanzette aus der Ebene des Trägerbandes herauszubewegen. Die Lanzette wird nach oder während dieses Knickvorgangs von einem Antriebselement in Richtung Gehäuseöffnung bewegt, um anschließend den Stechvorgang auszuüben. Dabei tritt mindestens ein Teil der Lanzette aus der Gehäuseöffnung aus und sticht in die Haut des Patienten. An der Einstichstelle bildet sich ein Bluttropfen, der zur Analyse benutzt wird. Wenn sich ein Testelement auf dem Trägerband befindet, wird das Trägerband, wenn nötig so weit transportiert, dass des Testelement sich unterhalb der Gehäuseöffnung befindet. Der Bluttropfen kann auf das Testelement aufgebracht werden, ohne dass der Patient weitere Schritte einleiten muss. Das Blut reagiert mit einem oder mehreren Reagenzien, die sich auf dem Testelement befinden, wie sie z. B. aus den Dokumenten EP-A 0 885 591, EP-B 0 535 480 und EP-B 0 477 322 bekannt sind. Das Testelement wird mittels eines Detektors analysiert.

Das Blut kann auf verschiedene Komponenten hin untersucht werden, wie es im Stand der Technik bekannt ist. Zum Beispiel kann die Analyse auf Blutbestandteile wie Hämatokrit, Glucose, Cholesterin, Koagulation, Eisen und andere gerichtet sein. Zur Analyse können unterschiedliche Methoden zur Anwendung kommen. So können beispielsweise elektrochemische Nachweisreaktionen benutzt werden, aber auch optische (z.B. Reflektion, Absorption, Fluoreszenz, Raman-Spektroskopie) oder magnetische Nachweisreaktionen. Typischerweise wird die Flüssigkeit mit einem Testsystem in Kontakt gebracht, wobei eine Reaktion zwischen einem Testelement und der Flüssigkeit stattfindet. So beruht die Detektion mittels eines optischen Testelements auf einer Farbreaktion zwischen Flüssigkeit und Nachweisreagenz. Beispiele für diese Reaktionen sind in den US Patenten 3,802,842; 4,061,468 und 4,490,465 beschrieben.

Bei der Benutzung des Gerätes führt das System verschiedene Schritte durch. Die Lanzette wird in eine Position gebracht, in der sie durch Einwirkung einer Schwellenkraft auf den Lanzettenkörper in den geknickten Zustand gebracht wird. Dabei wird vorzugsweise der Sterilschutz durchbrochen. Die Lanzette wird, wenn nötig, bis zur Öffnung des Gehäuses transportiert. Dort wird sie mit Hilfe eines Antriebselements aktuiert und tritt dabei zu einem Teil aus der Gehäuseöffnung aus. Bei dem Aktuationsvorgang tritt die Lanzette zumindest zu einem Teil in die Haut des Patienten ein und danach wieder in das Gerät zurück. Wenn das Transportband weiter transportiert und auf die zweite Spule gewickelt wird, liegt die Lanzette wieder flach auf dem Trägerband. Dieser Remagazinierungsvorgang wird in der Patentanmeldung US 20050245845 beschrieben.

In einem integrierten System, in dem auch Testelemente auf dem Trägeband, vorzugsweise alternierend mit den Lanzetten aufgebracht sind, wird das Testelement nach dem Stechvorgang bis zur Gehäuseöffnung transportiert, um den Bluttropfen zur Analyse aufzunehmen. Das Testelement kann bis zum Detektor transportiert und dort vermessen werden.

### Kurzbeschreibung der Figuren

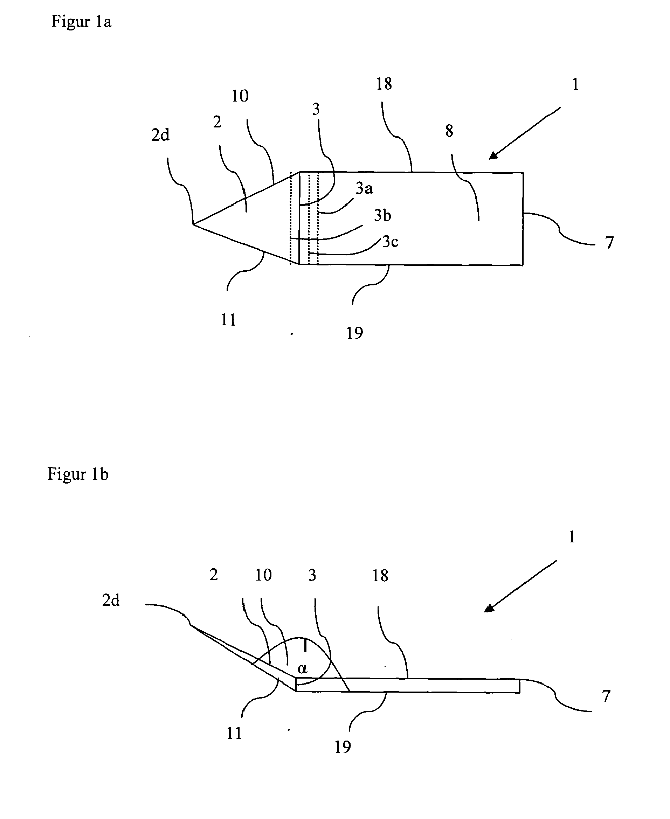

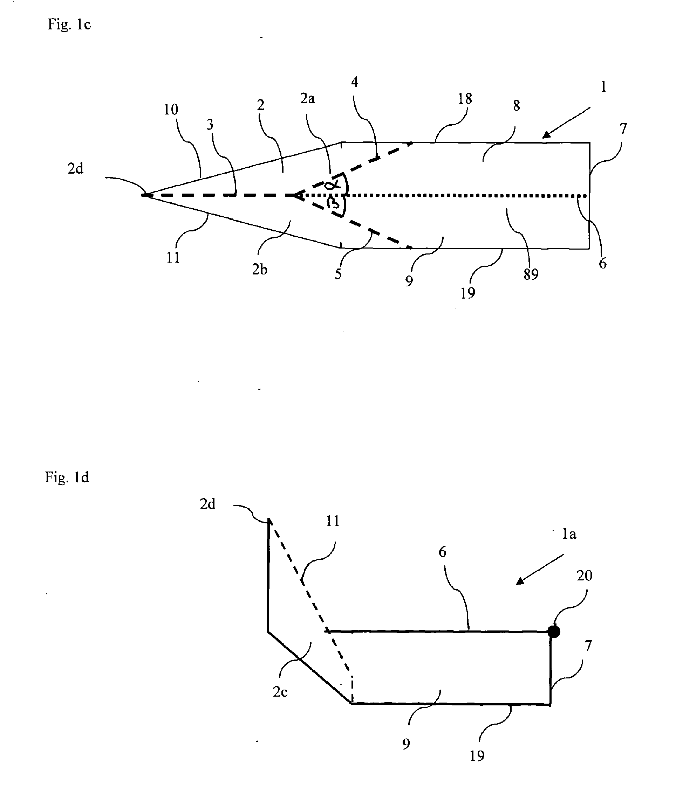

Figur 1 a: Schematische Darstellung einer Lanzette mit einer Prägung in unbenutztem Zustand.
Figur 1b: Schematische Darstellung einer Lanzette mit einer Prägung in geknicktem Zustand.
Figur 1c: Schematische Darstellung einer Flachlanzette mit mehreren Prägungen in unbenutztem Zustand.
Figur 1 d: Schematische Darstellung einer Lanzette mit mehreren Prägungen in geknicktem Zustand.
Figur 2: Schematische Darstellung der Queranordnung von Lanzetten auf dem Trägerband.
Figur 2a: Schematische Darstellung der Lanzetten in Längsanordnung auf dem Trägerband.
Figur 3: Schematische Darstellung von Lanzette und Band und deren Führung im Längsschnitt.
Figur 4a: Schematische Darstellung der Aktuation der Lanzette in dem das Trägerband über eine Rolle geführt wird.
Figur 4b: Schematische Darstellung der Lanzette in Aktuation wobei das Band horizontal aus seiner Ebene bewegt wird.
Figur 5a: Schematische Darstellung des Trägerbandes mit alternierender Anordnung von Testfeldern und längs angeordneten Lanzetten.
Figur 5b: Schematische Darstellungen des Trägerbandes mit alternierender Anordnung von Testfeldern und quer angeordneten Lanzetten.
Figur 6: Schematische Darstellung eines integrierten Gerätes mit Gehäuse und allen wichtigen Komponenten.

### Beschreibung der Figuren

Figur 1a stellt eine mögliche Ausführungsform der Lanzette (1) dar. Die Lanzette (1) besitzt ein distales Ende (2) und ein proximales Ende (7). Die Lanzette (1) besitzt einen Bereich (8), der sich an das proximale Ende (7) anschließt und in den Spitzenbereich (2) übergeht. Die Lanzette (1) weist eine Struktur (3) mit einer geänderten Steifigkeit auf, die im Folgenden als Prägung bezeichnet wird. Die Prägung (3) befindet sich im Knickbereich, der innerhalb oder außerhalb des Spitzenbereiches (2) liegen kann. Diese Prägung (3) kann sich an verschiedenen Stellen (3a), (3b), (3c) zwischen distalem und proximalem Ende der Lanzette befinden. Der Knickbereich wird durch den Bereich der Prägungen (3, 3a, 3b, 3c) definiert und kann je nach Anzahl der Prägungen variieren. Der Spitzenbereich (2) mündet in der Lanzettenspitze (2d). Beim Abknicken der Lanzette, bewirkt ein Knickelement das durch ein Regelelement gesteuert wird (hier nicht gezeigt, s. Fig. 7), dass die Lanzette an einer der Prägungen (3) bzw. (3a), (3b), (3c) abgeknickt wird. Je nach Lage des Knicks kann die Lanzette (1) beim Stechvorgang unterschiedlich tief in die Haut eindringen.

Eine Lanzette (1) mit einer Prägung (3) wird in Figur 1b im geknickten Zustand gezeigt. Die Lanzettenspitze(2d) sowie der Spitzenbereich (2) sind um den Winkel α zum Bereich (8) abgewinkelt. Der Winkel α kann zwischen 180° und ca. 80° liegen. Ein bevorzugter Bereich liegt zwischen 150° und 110°.

In Figur 1c wird eine Lanzette (1) dargestellt die eine bevorzugte Ausführungsform der Erfindung darstellt. Diese Lanzette (1) besitzt vorzugsweise mindestens eine Struktur mit einer geänderten Steifigkeit. Diese geänderte Steifigkeit dient zur leichteren und gezielten Veränderung der Ausrichtung der-Lanzettenspitze in Bezug auf den restlichen Lanzettenkörper. Bevorzugter weise ist die Steifigkeit in dieser Struktur erniedrigt, sodass bei Einwirkung einer Kraft auf den Lanzettenkörper, den Spitzenbereich (2) mit der Lanzettenspitze (2d) an der Stelle der erniedrigten Steifigkeit abknickt. Eine solche Struktur veränderter Steifigkeit kann durch verschiedene Methoden erreicht werden. So kann beim Herstellungsprozess der Lanzette (1) an dieser Stelle weniger Material eingebracht werden. Eine weitere Möglichkeit wäre ein Stanz- oder Hammerprozess zum Einbringen der Struktur oder eine Prägevorgang. Es sind weiterhin alle im Stand der Technik bekannten Metall verarbeitenden Methoden verwendbar, die zu einer Struktur mit veränderter Steifigkeit führen. In einer bevorzugten Struktur ist die erste Prägung (3) vom distalen Ende (2d) der Lanzette (1) zumindest über einen Teil des Spitzenbereiches (2) in Richtung des proximalen Endes (7) der Lanzette (1) in das Material eingeprägt. Diese Prägung (3) kann sich bis an das proximale Ende (7) der Lanzette erstrecken. Lateral von dieser Prägung aus kann eine zweite Prägung (4) in das Material eingebracht sein. Diese Prägung kann innerhalb oder außerhalb des Spitzenbereiches (2) beginnen, die sich von der Mitte (6) der Lanzette (1) in Richtung Seitenrand (18) erstreckt. Der Winkel zwischen dieser Prägung (4) und der Mittelinie (6) der Lanzette (1) α beträgt zwischen 0° und 90°. Bevorzugter Weise liegt dieser Winkel (α) zwischen 30° und 70°. Eine dritte Prägung (5) erstreckt sich auf der gegenüber liegenden Seite zu der Prägung (4). Auch diese Prägung (5) erstreckt sich von der Mitte (6) der Lanzette (1) hin zum Seitenrand (19). Der Winkel zwischen der Mittellinie (6) und der Prägung (5) liegt ebenfalls zwischen 0° und 90°, wobei ein bevorzugter Bereich zwischen 30° und 70° liegt. Die Winkel α und β müssen nicht identisch sein. Der Übergang der Seitenkanten (10) und (11) in die Seitenkanten (18) und (19) der Lanzette bilden die Grenze des Spitzenbereiches. Dabei treffen sich die beiden Seitenkanten (10) und (11) in der Spitze (2d).Der Knickbereich kann innerhalb, aber auch außerhalb des Spitzenbereiches (2) der Lanzette liegen und erstreckt sich in einer bevorzugten Ausführungsform über die ganze Länge der Lanzette (1). Um die Prägungen in eine Lanzette (1) einbringen zu können, wird vorzugsweise eine Flachlanzette benutzt, die aus einem dünnen Blech besteht.

Durch die Prägungen (3), (4), (5) in das Blech entstehen Knicklinien. Diese Knicklinien ergeben zumindest teilweise Knickung des Bleches zur einen und teilweise zur anderen Seite der Lanzettenebene (89) aus Figur 1c. Die Lanzettenebene (89) wird aus den Flächen (8) und (9) der ungeknickten Lanzette (1) gebildet. In Figur 1c liegt die Lanzettenebene (89) in der Papierebene. Die geknickte Lanzette ist in Figur 1d in der Seitenansicht dargestellt, sodass die Lanzettenebene (89) um 90° aus der Papierebene gedreht ist. Das gezielte Knicken der Lanzette (1) kann auch durch Perforation oder Ritzung oder Ätzung entlang der Linien (3), (4), (5) erreicht werden. Durch die spezielle Anordnung der Knicklinien (3), (4), (5) ergibt sich bei Krafteinwirkung auf die Lanzette ein Abknicken der Spitze (2d) bis über 90° zur Lanzettenfläche (89) und gleichzeitig ein Abknicken der Seitenflächen (2a) und (2b) des Spitzenbereiches (2) sowie ein entgegen gesetztes Abknicken der Flächen (8) und (9) des restlichen Lanzettenkörpers. Diese bevorzugte Ausführungsform weist eine hohe Stabilität des Lanzettenkörpers auf, trotz der Struktur mit erniedrigter Stabilität. Die Lanzette (1) ist ausreichend stabil, um einen Stechvorgang zur Blutgewinnung in die Haut eines Patienten durchzuführen. In ungeknickten Zustand, vor oder nach einem Stich, kann die Lanzette bevorzugter weise auf ein Band aufgerollt werden, wie in der Patentanmeldung US 20050245845 beschrieben. Der Stich kann zum Beispiel durch Drehung der Lanzette um das Lanzettenende (20) ausgeführt werden. Die Prägungen (3), (4) schließen eine Fläche (2a) ein, während die Prägungen (3), (5) eine Fläche (2b) einschließen. In dem Ausführungsbeispiel, dargestellt in Figur 1c enden die Prägungen (4), (5) außerhalb des Spitzenbereiches (2) der durch die Seitenkanten (10), (11) abgegrenzt wird. Diese Linien verlaufen über den Spitzenbereich hinaus in den Kanten (18), (19) bis zum proximalen Ende (7) der Lanzette (1). In einer bevorzugten Ausführungsform könnten zusätzlich zu den Prägungen (4) und (5) weitere Prägungen vorzugsweise parallel zu den Prägungen (4) und (5) in den Knickbereich eingearbeitet sein (nicht in den Figuren gezeigt). Mit Hilfe dieser alternativen Prägungen kann die Spitze an unterschiedlichen Stellen abgeknickt werden und dadurch unterschiedliche Längen aufweisen. Auf diese Weise können unterschiedliche Stechtiefen in die Haut ermöglicht werden.

In Figur 1 d ist die Lanzette in geknicktem Zustand dargestellt. In dieser Seitenansicht ist nun nur noch ein Teil der Flächen zu erkennen, bei dem Knickvorgang werden die Flächen (2a), (2b) nach oben aus der Lanzettenebene (89) gebogen während die Flächen (8), (9) sich nach unten aus der Ebene (89) bewegen. Die Mittellinie (6) bleibt dabei vorzugsweise in der Lanzettenebene (89). Die Knickung der Lanzettenspitze (2d) kann bis zu 100° zur Fläche der Lanzettenebene (89) stattfinden. Dabei wird in der seitlichen Ansicht die Unterseite des Spitzenbereiches (2) sichtbar, die in Figur 1 d als Fläche (2c) zu erkennen ist. Diese bildet die Rückseite der Fläche (2b) in Figur 1 c. In der Seitenansicht der Figur 1 d nicht zu erkennen, sind die Flächen (8) und (2a). Die Fläche (8) liegt hinter der Fläche (9), während sich Fläche (2a) hinter der Fläche (2c) versteckt. Bei Drehung der Lanzette (1) im Uhrzeigersinn um den Drehpunkt (20), führt die Lanzette (1) eine Bewegung nach oben und bei Rückdrehung nach unten aus. Ein alternativer Antrieb der Lanzette (1) wäre durch Ergreifen des Lanzettenschaftes am proximalen Ende (7) der Lanzette (1) mit einem Greifer oder einer Zange möglich. Hierbei würde die Lanzette (1) nicht um einen Punkt (20) gedreht sondern im Ganzen bewegt. In dieser Ausfixhrungsform sollte das Trägerband (14) eine ausreichende Flexibilität aufweisen, um die Bewegung der Lanzette nicht einzuschränken.

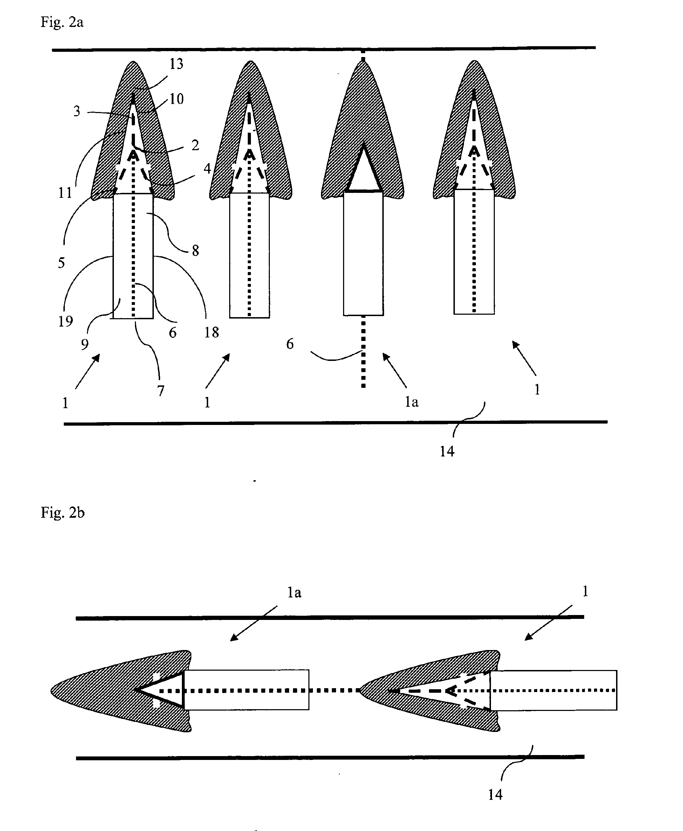

Figur 2a zeigt die Lanzetten (1) nach ihrer Herstellung. In dieser speziellen Ausführung sind die Lanzetten (1) durch Stanzen oder Ätzen aus einem dünnen Blechband, das hier das Trägerband (14) darstellt, herausgearbeitet. Die Lanzetten (1) sind auf dem Trägerband (14) quer zueinander angeordnet. Im Spitzenbereich (2) weisen sie die Prägungen (3), (4) und (5) auf, wobei die Prägungen (4) und (5) am proximalen Ende der Kanten (10) und (11) enden. Um den Spitzenbereich herum erstreckt sich ein Hohlraum (13), der durch Ausstanzen oder Ätzen gefertigt wird. Durch diesen Hohlraum (13) im Trägerband (14) um den Spitzenbereich (2) herum, kann die Spitze (2d) aus der Lanzettenebene herausgeknickt werden, wie dies für eine Lanzette (1a) dargestellt ist. Bei dieser Lanzette (1a) ist die Spitze (2d) nach oben geknickt, dabei stellt die Lanzettenmittellinie (6) die Biegelinie dar.

In Figur 2b sind die Lanzetten (1) auf dem Trägerband (14) in Längsausrichtung angeordnet. In Figur 2a ist sowohl eine ungeknickte Lanzette (1) als auch eine geknickte Lanzette (1a) dargestellt. Bei der geknickten Lanzette (1a) ist die Lanzettenspitze (2d) aus der Trägerbandebene (14) herausgeknickt.

Figur 3 ist eine schematische Darstellung der Anordnung von Lanzette (1) zu Trägerband (14) sowie zu einer Führung (15) des Trägerbandes (14). Die Führung (15) ist hier in einem gleichseitigen Dreieck dargestellt, wobei das Trägerband (14) über eine Kante (16) geleitet wird. Hierbei ist zu erkennen, dass die Lanzettenspitze (2d), in dem Moment, in dem die Lanzette (1) die Umlenkkante (16) erreicht, aus der Trägerbandebene herausgeknickt wird. Diese Möglichkeit Lanzetten zu knicken, ist besonders bevorzugt bei Lanzetten, wie sie in Figur 1c beschrieben werden, einzusetzen.

Figur 4a zeigt eine weitere Möglichkeit das Trägerband (14) so zu führen, dass die Lanzettenspitze (2d) automatisch aus der Trägerbandebene (14) herausgeknickt wird. In diesem Fall wird das Trägerband (14) über eine Rolle (21) geführt, die je nach Anordnung des Trägerbandes (14) auf der Rolle (21) entweder rechts oder links herum gedreht werden kann. In diesem Ausführungsbeispiel erstreckt sich die Lanzette (1) in Längsausrichtung auf dem Band (14) und das proximale Ende (7) der Lanzette (1) bewegt sich der Lanzettenspitze (2d) voraus. Die Rolle (21) kann beispielsweise aus einem profilierten Rad bestehen, das ein Rutschen des Trägerbandes (14) auf der Rolle (21) verhindert. Der Stechvorgang erfolgt hierbei durch schnelles Vor- und Zurückdrehen des Rades (21). Das Abknicken der Lanzette (1) kann hierbei durch ein zusätzliches Knickelement (z. B. eine Wulst, hier nicht dargestellt) auf dem Rad (21) erleichtert werden. Durch diese Wulst wird zusätzlich zur Kraft durch die Drehbewegung des Rades (21) eine Kraft auf die Mitte (6) des Lanzettenkörpers ausgeübt. Diese Kraft bewirkt das Knicken der Seitenflächen (8) und (9) des Lanzettenkörpers und damit das Abknicken der Lanzettenspitze (2d).

In Figur 4b ist eine geknickte Lanzette (1a) gezeigt, deren Spitze (2d) aus der Trägerbandebene (14a) herausragt. Um den Stechvorgang durchführen zu können, kann das Band (14) durch einen Bolzen (hier nicht dargestellt) auf der, der Lanzette (1a) gegenüber liegenden Seite, in Richtung Lanzettenspitze (2d) bewegt werden. Eine weitere Möglichkeit die Lanzette (1a) zu bewegen, ist mit Hilfe einer Zange (hier nicht dargestellt), die den Schaft der Lanzette (1a) ergreift und mittels einer auf und ab Bewegung den Stechvorgang durchführt. Eine bevorzugte Ausführungsform für diesen Stechvorgang ist es, dass Band (14) vor dem Stechvorgang zu spannen. Die Elastizität des Trägerbandes ist bevorzugter weise so zu wählen, dass es um die Stechtiefe (ca. 2 - 3 mm) ausgelenkt werden kann. Dabei kann mit Hilfe der Variation der Krafteinwirkung auf das Trägerband die Auslenkung des Trägerbandes verändert werden und somit die Stechtiefe (bzw. Austrittsweite der Lanzette) variiert werden.

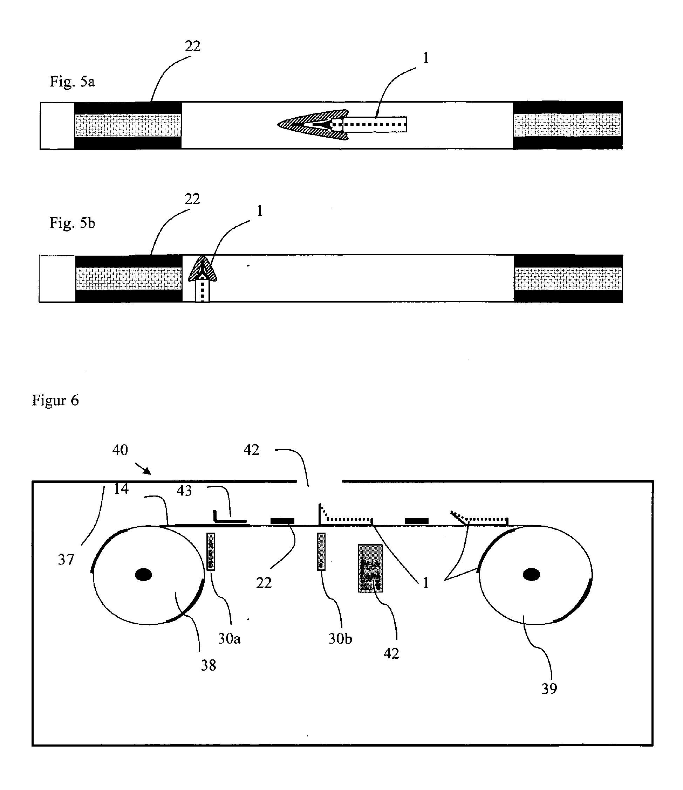

In Figur 5a wird ein Trägerband (14) gezeigt, auf dem alternierend ein Testfeld (22) und eine Lanzette (1) angeordnet sind. Dabei ist die Lanzette (1) in Längsausrichtung zum Trägerband angebracht. Der Abstand zwischen dem Testfeld (22) und der Lanzette (1) auf dem Trägerband (14) kann variieren. So ist es möglich, die Lanzette (1) so dicht neben dem Testfeld (22) zu platzieren, dass nach dem erfolgten Einstich die Flüssigkeit sofort von dem Testfeld (22) aufgenommen werden kann, ohne das Trägerband (14) zu bewegen. Eine weitere Ausführungsform mit alternierenden Testfeldern (22) und Lanzetten (1) ist in Abbildung 5b gezeigt. Hierbei ist die Lanzette (1) quer auf dem Trägerband angeordnet. Auch hier kann die Lanzette (1) in variablem Abstand zum Testfeld (22) platziert werden.

In Figur 6 ist ein integriertes System gezeigt. Das System besteht aus einem Gerät (40), das bevorzugter weise ein Gehäuse (37) mit einer Öffnung (41) sowie ein Trägerband (14), auf dem die Lanzetten (1) befestigt sind, besitzt. Das Trägerband (14) ist auf 2 Spulen (38) und (39) aufgewickelt. Dabei befindet sich der unbenutzte Teil der auf dem Trägerband befestigten Lanzetten auf der Spule (38) und der benutzte Teil wird auf die Spule (39) gewickelt. Zwischen den Spulen (38, 39) ist das Trägerband (14) gestreckt. Die Spulen (38, 39) werden durch einen Antrieb bewegt wie er aus dem Stand der Technik bekannt ist. Bevorzugter weise wird nur eine der beiden Spulen (38, 39) angetrieben. Ein Beispiel für einen solchen Antrieb ist in der Anmeldung US 20050245845 beschrieben. Die Lanzetten (1) befinden sich im ungeknickten Zustand auf dem Trägerband (14), wenn das Trägerband (14) auf den Spulen gewickelt ist. Zwischen den beiden Spulen (38, 39) befindet sich ein erster Stößel (30a), der dazu dient die Kraft für den Knickvorgang auf die Lanzette (1) zu übertragen. Dieser Stößel (30a) ist unterhalb des Trägerbandes (14) angeordnet. Damit die Kraft nicht ausschließlich zur Auslenkung des Trägerbandes (14) dient, befindet sich oberhalb des Trägerbandes (14) gegenüber dem Stößel (30a) ein Knickelement (43), das das Trägerband (14) an einer vertikalen Bewegung hemmt. Das Knickelement (43) kann, je nach Anordnung der mindestens einen Prägung auf der Lanzette, unterschiedliche Formen aufweisen. Bei einer Lanzette mit nur einer Prägung ist keine besondere Form des Knickelements nötig, da das Knickelement in dieser Ausführungsform ausschließlich die Funktion hat, die Lanzette mit dem Trägerband an einer weiteren Bewegung zu hindern. In einer bevorzugten Ausführungsform des Knickelements (43) bei der eine Lanzette mit mehr als einer Prägung geknickt werden soll, wie sie in Figur 1 c dargestellt ist, weist das Knickelement (43) eine Form auf, bei der die abzuknickenden Flächen (8) und (9) zwar an einer Bewegung gehindert werden, aber der Knickbereich mit der mindestens einen Prägung (6) nicht. Ein Beispiel für diese Anordnung ist ein Knickelement (43) mit zwei Flügeln, die sich über die abzuknickenden Flächen erstrecken, aber zwischen den Flügeln genug Raum bleibt, dass die Lanzette sich in diesen Raum weiter bewegen kann und geknickt wird.

Das Knickelement (43) kann ein Regelelement beinhalten (hier nicht gezeigt), das die Position des Knickelementes (43) so verändern kann, dass die Lanzette an unterschiedlichen Stellen geknickt wird. Dies ist besonders bevorzugt, bei Ausführungsformen, die nur eine Prägung (3) bzw. (3a, 3b, 3c) zum Abknicken der Lanzettenspitze benutzen.

Ein zweiter Stößel (30b), der sich unterhalb der Gehäuseöffnung (41) befindet, dient dazu die Lanzette bei der Aktuation anzutreiben. Zwischen Gehäuseöffnung (41) und Stößel (30b) befindet sich das Trägerband (14). Zum Auslösen des Stechvorgangs wird das Trägerband (14) soweit transportiert, bis eine unbenutzte Lanzette (1) zwischen Gehäuseöffnung (41) und Stößel (30b) liegt. Beim Auslösen des Stechvorgangs wird der Stößel (30b) mit so viel Kraft auf die Lanzette (1) zu bewegt, dass mindestens die Lanzettenspitze (2) aus der Gehäuseöffnung (41) heraus bewegt wird. Nach erfolgtem Einstich wird das Blut auf einem Testfeld (22) gesammelt. Dort findet eine Reaktion des Blutes mit den Reagenzien auf dem Testfeld statt, die mit Hilfe eines Detektors (42) analysiert werden kann. Die Lanzette (1) wird zusammen mit dem Trägerband (14) remagaziniert. Durch den Wicklungsvorgang auf der Spule (39) wird die Lanzette wieder flach in das Trägerband (14) integriert.

## Patentansprüche

1. Eine Vorrichtung zur Gewinnung von Körperflüssigkeit, beinhaltend:
ein im Wesentlichen planares Trägerband,
mindestens eine Lanzette, beinhaltend einen Lanzettenkörper und eine Spitze, wobei die Lanzette liegend auf dem Trägerband angeordnet ist, **dadurch gekennzeichnet, dass** die Lanzette einen Knickbereich beinhaltet, sodass die Lanzette unter Krafteinwirkung bevorzugt in dem Bereich der Spitze geknickt wird, sodass die Spitze in Bezug auf die Längsachse des Lanzettenkörpers in ihrer Ausrichtung verändert wird.

2. Vorrichtung nach Anspruch 1, **dadurch gekennzeichnet, dass** die mindestens eine Lanzette eine Flachlanzette ist.

3. Vorrichtung nach den Ansprüchen 1 oder 2, **dadurch gekennzeichnet, dass** beim Aufwenden einer Kraft auf den Lanzettenkörper, die Lanzettenspitze aus der im Wesentlichen planaren Ebene des Trägerbandes herausragt.

4. Vorrichtung nach einem der Ansprüche 1 bis 3, **dadurch gekennzeichnet, dass** die Lanzette im Knickbereich mindestens eine Struktur mit einer geänderten Steifigkeit besitzt.

5. Vorrichtung nach Anspruch 4, **dadurch gekennzeichnet, dass** sich mindestens eine Struktur mit geänderter Steifigkeit über einen Teil der Längsausdehnung des Spitzenbereiches erstreckt.

6. Vorrichtung nach einem der Ansprüche 4 bis 5, **dadurch gekennzeichnet, dass** bei Einwirkung einer Schwellenkraft auf die Lanzette, die mindestens eine Struktur mit veränderter Steifigkeit, ein Abknicken der angrenzenden Flächen bewirkt.

7. Vorrichtung nach einem der Ansprüche 1 bis 6, **dadurch gekennzeichnet, dass** die mindestens eine Lanzette in verschiedenen Ausrichtungen auf dem Trägerband angeordnet ist.

8. Vorrichtung nach einem der vorherigen Ansprüche, **dadurch gekennzeichnet, dass** auf dem Trägerband weiterhin mindestens ein Testelement angeordnet ist.

9. Vorrichtung nach einem der vorherigen Ansprüche, **dadurch gekennzeichnet, dass** die Lanzette an ihrem proximalen Ende auf dem Trägerband fixiert ist.

10. Vorrichtung nach einem der vorherigen Ansprüche, **dadurch gekennzeichnet, dass** mindestens die Lanzettenspitze einen Sterilschutz besitzt.

11. Vorrichtung nach Anspruch 10, **dadurch gekennzeichnet, dass** der Sterilschutz vor oder während der Benutzung der Lanzette von der Lanzettenspitze getrennt wird.

12. System zur Gewinnung von Körperflüssigkeit, bestehend aus
einem im Wesentlichen planaren Trägerband, und
mindestens einer Lanzette, die liegend auf dem Trägerband angeordnet ist, und
einer Lanzettenspitze die sich am distalen Ende der Lanzette befindet,
einem Knickelement das auf die Lanzette einwirkt, sodass die Lanzettenspitze unter Krafteinwirkung in Bezug auf die Längsachse der Lanzette in ihrer Ausrichtung veränderbar ist.

13. System nach Anspruch 12, mit einer Lanzette nach einem der Ansprüche 2 bis 11.

14. Vorrichtung nach Anspruch 12, **dadurch gekennzeichnet, dass** das Knickelement ein Regelelement beinhaltet, so dass die Lanzette an verschiedenen Stellen im Knickbereich geknickt werden kann.
